# EUROPEAN PATENT APPLICATION

(11) **EP 4 578 872 A1**
(43) Date of publication of application: **02.07.2025**
(21) Application number: 22956202.0
(22) Date of filing: 20.09.2022
(51) Int. Cl.: C07K 16/18, C07K 16/28, A61K 39/395, C12N 15/86

(54) **ANTI-TREM2 SINGLE-DOMAIN ANTIBODY AND USE THEREOF**

(30) Priority: 26.08.2022 CN 202211032031
(71) Applicant: Regenecore Biotech Co., Ltd, Nanjing, Jiangsu 210000 (CN)
(72) Inventor: SU, Zhipeng, Nanjing, Jiangsu 210000 (CN); WANG, Yang, Nanjing, Jiangsu 210000 (CN); XIE, Wei, Nanjing, Jiangsu 210000 (CN)
(74) Representative: Murgitroyd & Company
(86) International application number: PCT/CN2022/119860
(87) International publication number: WO 2024/040658

(57) **Abstract**

Provided is an anti-TREM2 single-domain antibody, consisting of heavy chains comprising CDR1 represented by any one of SEQ ID NOs: 34-40, CDR2 represented by any one of SEQ ID NOs: 41-45, and CDR3 represented by any one of SEQ ID NOs: 46-50. The single-domain antibody has good affinity with TREM2.

## Description

### TECHNICAL FIELD

The present disclosure relates to a single-domain antibody that can specifically bind to TREM2 (hereinafter abbreviated as "anti-TREM2 single-domain antibody"), a pharmaceutical composition containing the single-domain antibody as an active ingredient, and therapeutic use thereof.

### BACKGROUND

TREM2 is a transmembrane receptor of the immunoglobulin superfamily, which binds to the ligand DAP12 to transmit intracellular signals. DAP12, also referred to as TYRO protein tyrosine kinase binding protein (TYROBP), is a signaling adaptor protein expressed in cells involved in innate immune responses, which mediates the activation of spleen tyrosine kinase SYK. TREM2 may also bind to DAP10. DAP10 recruits phosphatidylinositol 3-kinase (PI3K) to promote signaling. Studies have shown that a TREM2-DAP12/DAP10 heterodimer may be formed, and the downstream signaling is very dependent on such a combination. TREM2 can be cleaved from a cell surface by a disintegrin, metalloproteinase structural-domain protein 10 (ADAM10) and γ-secretase, to release soluble TREM2 (sTREM2).

Currently, Alzheimer's disease, obesity-related metabolic syndrome, and cancer are the leading causes of death in humans and are also the three most costly diseases in the medical field in the Western world. In recent years, TREM2 has been recognized as a major pathology-induced immune signaling hub that plays an important role in the activation and survival of myeloid cells. Encoding variation on TREM2 increases the risk of Alzheimer's disease and other neurodegenerative diseases. TREM2 is also expressed by tumor-infiltrating macrophages. Anti-TREM2 monoclonal antibodies inhibit tumor growth and promote tumor regression when used in combination with anti-PD-1. There is increasing evidence that TREM2 plays a role in tumor-associated macrophages (TAMs) and myeloid-derived suppressor cells (MDSCs). A study found that, compared with a healthy control group, TREM2 was significantly upregulated in peripheral blood monocytes and TAMs in patients with lung cancer and mice with tumors transplanted. A TREM2 level of macrophages surrounding tumor cells is positively correlated with tumor progression. In addition, TREM2+ myeloid cells have a strong inhibitory effect on T cell proliferation in vitro. Increasing evidence indicates that TREM2 plays an important role in promoting the immunosuppressive tumor microenvironment.

One of the most prominent mechanisms of action of TREM2 as a therapeutic target is directly targeting an active region of a receptor using specific antibodies or small molecules to block or activate downstream signals. Therefore, the preparation of an antibody that can specifically identify and bind to TREM2 is of great importance in the diagnosis, treatment, and prognosis of TREM2-related diseases. Currently, anti-TREM2 single-domain antibody products with high affinity and medicinal value are still lacking in the related art.

### SUMMARY

An objective of the present disclosure is to provide a single-domain antibody that can specifically bind to TREM2, and use thereof.

According to a first aspect of the present disclosure, an anti-TREM2 single-domain antibody is provided. The single-domain antibody includes a heavy chain. The heavy chain includes a heavy chain CDR1 as set forth in any one of SEQ ID NO: 34 to SEQ ID NO: 40, a heavy chain CDR2 as set forth in any one of SEQ ID NO: 41 to SEQ ID NO: 45, and a heavy chain CDR3 as set forth in any one of SEQ ID NO: 46 to SEQ ID NO: 50.

Preferably, amino acid sequences of the heavy chain CDR1, the heavy chain CDR2, and the heavy chain CDR3 are one of the following (1) to (8):
(1) CDR1 as set forth in SEQ ID NO: 34, CDR2 as set forth in SEQ ID NO: 42, and CDR3 as set forth in SEQ ID NO: 49;
(2) CDR1 as set forth in SEQ ID NO: 35, CDR2 as set forth in SEQ ID NO: 41, and CDR3 as set forth in SEQ ID NO: 50;
(3) CDR1 as set forth in SEQ ID NO: 35, CDR2 as set forth in SEQ ID NO: 41, and CDR3 as set forth in SEQ ID NO: 49;
(4) CDR1 as set forth in SEQ ID NO: 36, CDR2 as set forth in SEQ ID NO: 44, and CDR3 as set forth in SEQ ID NO: 48;
(5) CDR1 as set forth in SEQ ID NO: 37, CDR2 as set forth in SEQ ID NO: 44, and CDR3 as set forth in SEQ ID NO: 48;
(6) CDR1 as set forth in SEQ ID NO: 38, CDR2 as set forth in SEQ ID NO: 43, and CDR3 as set forth in SEQ ID NO: 46;
(7) CDR1 as set forth in SEQ ID NO: 39, CDR2 as set forth in SEQ ID NO: 45, and CDR3 as set forth in SEQ ID NO: 47; and
(8) CDR1 as set forth in SEQ ID NO: 40, CDR2 as set forth in SEQ ID NO: 45, and CDR3 as set forth in SEQ ID NO: 47.

The CDR combinations (1) - (7) correspond to the single-domain antibodies 10F10, 2B5, 12G10, 10E12, 11H2, 1H1, and 11D3, respectively. The CDR combination (8) corresponds to the single-domain antibodies 10B3 and 12G12W.

Each of the foregoing sequences may be substituted by a sequence having "at least 80% homology" with each of the foregoing sequences or a sequence with only one or a few amino acid substitutions, preferably "at least 85% homology", more preferably "at least 90% homology", more preferably "at least 95% homology", and most preferably "at least 98% homology".

In an embodiment, one to five arbitrary amino acid residues of any one or more CDRs of the heavy chain CDR1, CDR2, and CDR3 may be respectively substituted by conserved amino acids thereof. Specifically, one to five amino acid residues of the heavy chain CDR1 may be substituted by conserved amino acids thereof; one to five amino acid residues of the heavy chain CDR2 may be substituted by conserved amino acids thereof; and one to five amino acid residues of the heavy chain CDR3 may be substituted by conserved amino acids thereof.

As used herein, the term "sequence homology" means the extent to which two (nucleotide or amino acid) sequences have the same residues at the same positions in alignment, and is usually expressed as a percentage. Preferably, homology is determined over the overall length of the aligned sequences. Therefore, two copies with identical sequences have 100% homology.

In some embodiments, a sequence with only one or a few amino acids substituted, for example, including 1, 2, 3, 4, 5, 6, 7, 8, 9, or 10 conserved amino acid substitutions, with respect to the foregoing sequences, can also achieve the purpose of the present disclosure. These forms of variation include (but are not limited to): one or more (usually 1 to 50, preferably 1 to 30, more preferably 1 to 20, or most preferably 1 to 10) amino acid deletions, insertions, and/or substitutions, and addition of one or more (usually less than 20, preferably less than 10, or more preferably less than 5) amino acids at the C-terminus and/or N-terminus. Actually, when the extent of sequence homology between two amino acid sequences is determined or when the combination of CDR1, CDR2, and CDR3 in the single-domain antibody is determined, a person skilled in the art may take into account the so-called "conserved" amino acid substitution. In the case of substitutions, the substitutions are preferably conserved amino acid substitutions. The conserved amino acid substitutions may usually be described as an amino acid substitution in which an amino acid residue is substituted by another amino acid residue having a similar chemical structure, without little or no effect on the function, activity, or other biological properties of the polypeptide. The conserved amino acid substitution is common in the art. For example, the conserved amino acid substitutions are a substitution of one or a few amino acids in the following groups (a)-(d) by another one or a few amino acids in the same groups: (a) polar negatively-charged residues and uncharged amides thereof: Asp, Asn, Glu, and Gln; (b) polar positively-charged residues: His, Arg, and Lys; (c) aromatic residues: Phe, Trp, and Tyr; and (d) aliphatic non-polar or weakly-polar residues: Ala, Ser, Thr, Gly, Pro, Met, Leu, Ile, Val, and Cys. Particularly, the preferred conserved amino acid substitutions are as follows: Asp is substituted by Glu; Asn is substituted by Gln or His; Glu is substituted by Asp; Gln is substituted by Asn; His is substituted by Asn or Gln; Arg is substituted by Lys; Lys is substituted by Arg or Gln; Phe is substituted by Met, Leu, or Tyr; Trp is substituted by Tyr; Tyr is substituted by Phe or Trp; Ala is substituted by Gly or Ser; Ser is substituted by Thr; Thr is substituted by Ser; Gly is substituted by Ala or Pro; Met is substituted by Leu, Tyr, or Ile; Leu is substituted by Ile or Val; Ile is substituted by Leu or Val; Val is substituted by Ile or Leu; or Cys is substituted by Ser. In addition, a person skilled in the art knows that framework region sequences FR1-4 are not immutable, and the sequences FR1-4 may be conserved sequence variants of the sequences disclosed in the present disclosure.

The meaning of "anti-TREM2 single-domain antibody" of the present disclosure includes not only an intact single-domain antibody, but also fragments, derivatives, and analogs of the anti-TREM2 single-domain antibody. As used herein, the terms "fragments", "derivatives", and "analogs" have the same meaning, which indicate a polypeptide with substantially the same biological function or activity as the antibody of the present disclosure. The polypeptide fragments, derivatives, or analogs of the present disclosure may be (i) a polypeptide with one or more conserved or non-conserved amino acid residues (preferably conserved amino acid residues) substituted, where such substituted amino acid residues may or may not be encoded by the genetic code, (ii) a polypeptide having a substituent in one or more amino acid residues, (iii) a polypeptide formed by fusing a mature polypeptide with another compound (for example, a compound that extends the half-life of the polypeptide, such as polyethylene glycol), or (iv) a polypeptide (such as a leading sequence or a secretory sequence, or a sequence for purifying this polypeptide or a proteogenic sequence, or a fusion protein formed with an Fc tag) formed by fusing an additional amino acid sequence to this polypeptide sequence. According to this specification, these fragments, derivatives, and analogs fall within the scope of common general knowledge of a person skilled in the art.

In a preferred embodiment, the heavy chain further includes a framework region FR; the framework region FR includes amino acid sequences of framework region 1 (FR1), framework region 2 (FR2), framework region 3 (FR3), and framework region 4 (FR4); and the amino acid sequences of the framework region FR are as follows respectively:
FR1 as set forth in SEQ ID NO: 19 or SEQ ID NO: 20, or an FR1 variant including at most five amino acid substitutions with respect to FR1;
FR2 as set forth in any one of SEQ ID NO: 21 to SEQ ID NO: 25, or an FR2 variant including at most five amino acid substitutions with respect to FR2; and
FR3 as set forth in any one of SEQ ID NO: 26 to SEQ ID NO: 32, or an FR3 variant including at most five amino acid substitutions with respect to FR3; and
FR4 as set forth in SEQ ID NO: 33, or an FR4 variant including at most five amino acid substitutions with respect to FR4.

According to a second aspect of the present disclosure, an amino acid sequence that can bind to an anti-TREM2 single-domain antibody is provided. The amino acid sequence of the single-domain antibody is as set forth in SEQ ID NO: 1 to SEQ ID NO: 9. respectively, or the single-domain antibody has at least 80% sequence homology with the amino acid sequences as set forth in any one of SEQ ID NO: 1 to SEQ ID NO: 9, and can specifically bind to the TREM2 protein.

In an embodiment, the anti-TREM2 single-domain antibody is selected from SEQ ID NO: 1 to SEQ ID NO: 9 or has at least 90%, 91%, 92%, 93%, 94%, 95%, 96%, 97%, 98%, 99%, or 100% sequence homology with the amino acid sequence selected from any one of SEQ ID NO: 1 to SEQ ID NO: 9, and can specifically bind to the TREM2 protein.

According to a third aspect of the present disclosure, an Fc fusion antibody or a humanized antibody of the foregoing any anti-TREM2 single-domain antibody is provided.

According to a fourth aspect of the present disclosure, a nucleotide molecule for encoding the foregoing anti-TREM2 single-domain antibody, the foregoing Fc fusion antibody, or the foregoing humanized antibody is provided. A nucleotide sequence of the nucleotide molecule is as set forth in SEQ ID NO: 10 to SEQ ID NO: 18, respectively, or has at least 95% sequence homology with any one of SEQ ID NO: 10 to SEQ ID NO: 18.

In an embodiment, the nucleic acid molecule for encoding the anti-TREM2 single-domain antibody is selected from SEQ ID NO: 10 to SEQ ID NO: 18, or has at least 90%, 91%, 92%, 93%, 94%, 95%, 96%, 97%, 98%, 99%, or 100% sequence homology with the nucleotide sequences selected from any one of SEQ ID NO: 10 to SEQ ID NO: 18, and the anti-TREM2 single-domain antibody encoded by the nucleic acid molecule can specifically bind to the TREM2 protein.

According to a fifth aspect of the present disclosure, an expression vector is provided, including a nucleotide molecule for encoding the anti-TREM2 single-domain antibody, or the Fc fusion antibody or the humanized antibody thereof. The nucleotide sequence is as set forth in SEQ ID NO: 10 to SEQ ID NO: 18, respectively, or has at least 80% sequence homology with any one of SEQ ID NO: 10 to SEQ ID NO: 18.

In a preferred embodiment, the used expression vector is RJK-V4-3 or RJK-V4-hFC1 (the nucleotide molecule for encoding the anti-TREM2 single-domain antibody or the Fc fusion antibody or the humanized antibody thereof is integrated into RJK-V4-3 or RJK-V4-hFC1 by means of genetic engineering). Another universal expression vector may be alternatively selected as needed.

According to a sixth aspect of the present disclosure, a host cell that can express the foregoing anti-TREM2 single-domain antibody, or the Fc fusion antibody, or the humanized antibody thereof is provided, or includes the foregoing expression vector. Preferably, the host cell is a bacterial cell, a fungal cell, or a mammalian cell.

In another preferred embodiment, the host cell includes a prokaryotic cell or a eukaryotic cell, including bacteria or fungi.

In another preferred embodiment, the host cell is selected from the following group: *Escherichia coli,* a yeast cell, a mammalian cell, a phage, or a combination thereof.

In another preferred embodiment, the prokaryotic cell is selected from the following group: *Escherichia coli, Bacillus subtilis, Lactobacillus, Streptomyces, Proteus mirabilis,* or a combination thereof.

In another preferred embodiment, the eukaryotic cell is selected from the following group: *Pichia pastoris, Saccharomyces cerevisiae,* fission yeast, *Trichoderma,* or a combination thereof.

In another preferred embodiment, the eukaryotic cell is selected from the following group: cells from insects such as *Spodoptera frugiperda,* cells from plants such as tobacco, BHK cells, CHO cells, COS cells, myeloma cells, or a combination thereof.

In another preferred embodiment, the host cell is ExpiCHO-S cells in suspension.

In another preferred embodiment, the host cell is 293F cells in suspension.

According to a seventh aspect of the present disclosure, a recombinant protein is provided, including the foregoing anti-TREM2 single-domain antibody. The recombinant protein may be the single-domain antibody as set forth in any one of SEQ ID NO: 1 to SEQ ID NO: 9, or the single-domain antibody having at least 80% homology with any one of SEQ ID NO: 1 to SEQ ID NO: 9, or may be a multi-epitope antibody, a multi-specific antibody, and a multivalent antibody. For example, the multi-epitope antibody may consist of more than one sequence of SEQ ID NO: 1 to SEQ ID NO: 9; the multivalent antibody may consist of repeatedly arranging one sequence of SEQ ID NO: 1 to SEQ ID NO: 9; and the multi-specific antibody includes but is not limited to a bispecific antibody and a trispecific antibody. In addition, the recombinant protein may be a fragment, derivative, and analog of the foregoing antibody.

According to an eighth aspect of the present disclosure, a pharmaceutical composition is provided, including the foregoing anti-TREM2 single-domain antibody and a pharmaceutically acceptable carrier. Usually, these substances may be prepared in a non-toxic, inert, and pharmaceutically acceptable aqueous carrier medium with a pH usually determined based on the isoelectric point of the antibody (the pH of the aqueous carrier medium deviates from the isoelectric point of the antibody and differs from the isoelectric point of the antibody by about 2).

The pharmaceutical composition of the present disclosure can be directly used to bind to TREM2 protein molecules, and therefore can be used to treat a neurodegenerative disease, more preferably, Alzheimer's disease. In addition, the pharmaceutical composition may be used in combination with another therapeutic agent for Alzheimer's disease.

The pharmaceutical composition of the present disclosure may also be used to treat a tumor. In addition, another therapeutic agent for the tumor may be used concurrently in combination.

The pharmaceutical composition in the present disclosure contains a safe and effective amount (for example, 0.001 wt% to 99 wt%, preferably 0.01 wt% to 90 wt%, more preferably 0.1 wt% to 80 wt%) of the foregoing single-domain antibody, and a pharmaceutically acceptable carrier or excipient. Such carrier includes (but is not limited to): saline, buffer, glucose, water, glycerol, ethanol, and a combination thereof. A pharmaceutical preparation may suit a mode of administration. The pharmaceutical composition of the present disclosure may be prepared in the form of an injection, for example, prepared by a conventional method with saline or an aqueous solution containing glucose and other adjuvants. The pharmaceutical composition such as an injection or a solution is desirably prepared under a sterile condition.

According to a ninth aspect of the present disclosure, a medicament for treatment of Alzheimer's disease or a tumor is provided, including the foregoing single-domain antibody for binding to the TREM2 protein as an active ingredient.

According to a tenth aspect of the present disclosure, a kit for detecting a TREM2 level is provided, including the foregoing anti-TREM2 single-domain antibody. In a preferred embodiment of the present disclosure, the kit further includes a container, an instruction, a buffer, etc.

In a preferred embodiment, the kit includes an antibody for identifying the TREM2 protein, a lysing matrix for lysing a sample, and a general reagent and buffer required for the detection, such as various buffers, detection labels, and detection substrates. The detection kit may be an in vitro diagnostic device.

In a preferred embodiment, the kit further includes a secondary antibody, an enzyme, a fluorescent agent or a radioactive label for detection, and a buffer.

In a preferred embodiment, the secondary antibody of the kit may be an antibody (as an anti-antibody) against the anti-TREM2 single-domain antibody, which may be a single-domain antibody, a monoclonal antibody, a polyclonal antibody, or any other form of the antibody.

According to an eleventh aspect of the present disclosure, a method for producing the anti-TREM2 single-domain antibody is provided, including the following steps:
(a) culturing the host cell according to the sixth aspect of the present disclosure under a condition suitable for producing the single-domain antibody, to obtain a culture containing the anti-TREM2 single-domain antibody; and
(b) isolating or recovering the anti-TREM2 single-domain antibody from the culture; and
(c) optionally, purifying and/or modifying the anti-TREM2 single-domain antibody obtained in step (b).

According to a twelfth aspect of the present disclosure, use of the foregoing anti-TREM2 single-domain antibody or the foregoing pharmaceutical composition in preparation of a medicament for treatment of a neurodegenerative disease.

In a preferred embodiment, the disease is Alzheimer's disease.

In a preferred embodiment, the disease is a tumor. For example, the anti-TREM2 single-domain antibody of the present disclosure plays a role in tumor-associated macrophages (TAMs) and myeloid-derived suppressor cells (MDSCs).

### Beneficial Effects

Compared with the related art, the present disclosure has the following beneficial effects:
(1) The single-domain antibody of the present disclosure specifically targets the TREM2 protein with a correct spatial structure.
(2) The single-domain antibody obtained in the present disclosure has a flexible choice among expression systems, which can be expressed in a prokaryotic system or in a eukaryotic system, such as yeast cells or mammalian cells, and has low expression costs when expressed in a prokaryotic expression system, which can reduce subsequent production costs.
(3) The single-domain antibody obtained in the present disclosure is simple in modifying as multiple combination forms of the antibody, which can obtain multivalent or multi-specific antibodies through simple tandem linkage by means of genetic engineering, and has very low immune heterogeneity, without a strong immune response even when subjected to no humanized modification.
(4) The single-domain antibody obtained in the present disclosure has a wider affinity range, which can vary from the nM level to the pM level before affinity maturation, providing multiple options for antibodies for subsequent different purposes.

### BRIEF DESCRIPTION OF THE DRAWINGS

To describe the technical solutions of this application more clearly, the following briefly describes the accompanying drawings required in the embodiments. Apparently, a person of ordinary skill in the art may further derive other drawings from these accompanying drawings without creative efforts.
FIG. 1 shows library enrichment of TREM2-targeted antibody screened according to Example 3;
FIG. 2, FIG. 3, FIG. 4, FIG. 5, FIG. 6, and FIG. 7 all are determination plots of antigen-binding dose-response curves of the antibody according to Example 12; and
FIG. 8, FIG. 9, FIG. 10, FIG. 11, FIG. 12, FIG. 13, FIG. 14, FIG. 15, and FIG. 16 all show the results of stimulation of antibodies (eukaryotic samples) on the proliferation of macrophages according to Example 13.

### DETAILED DESCRIPTION OF THE EMBODIMENTS

The present disclosure is further described in detail below with reference to embodiments, so as to enable a person skilled in the art to implement the present disclosure with reference to the text of this specification.

As used herein, the "single-domain antibody" (sdAb, also referred to as nanobody or variable domain of heavy chain of heavy-chain antibody (VHH) by the developer Ablynx) is known to a person skilled in the art. The single-domain antibody is an antibody whose complementarity determining region is a part of a single-domain polypeptide. Therefore, the single-domain antibody contains a single complementarity determining region (a single CDR1, a single CDR2, and a single CDR3). Examples of the single-domain antibody are a heavy-chain-only antibody (which naturally contains no light chains), a single-domain antibody derived from a conventional antibody, and an engineered antibody.

The single-domain antibody may be derived from any species, including mice, humans, camels, llamas, goats, rabbits, and cattle. For example, naturally occurring VHH molecules may be derived from antibodies provided by camelid species (such as camels, dromedary camels, llamas, and guanacos). Like an intact antibody, the single-domain antibody can selectively bind to a specific antigen. The single-domain antibody may contain only a variable domain of an immunoglobulin chain, and the domain has CDR1, CDR2, and CDR3 as well as framework regions.

As used herein, the term "Fc fusion antibody" refers to a novel protein produced by fusing an Fc segment of a target antibody with a biologically active functional protein molecule by the genetic engineering technique.

The term "humanized antibody" refers to an antibody obtained by fusing a heavy-chain variable region of a target antibody (such as an animal antibody) with a constant region of a human antibody, or an antibody obtained by transplanting complementarity determining regions (CDR1 to 3 sequences) of a target antibody into a variable region of a human antibody, or an antibody obtained by mutating amino acids of a target antibody based on features of framework regions (FR1 to 4) of a human antibody. The humanized antibody may be obtained by a synthetic method or a site-directed mutagenesis method.

In the present disclosure, the anti-TREM2 single-domain antibody may also be obtained from sequences having high homology with the sequences of CDR1 to 3 disclosed by the present disclosure. In some embodiments, sequences having "at least 80% homology", "at least 85% homology", "at least 90% homology", "at least 95% homology", or "at least 98% homology" with the sequences of any one of SEQ ID NO: 1 to SEQ ID NO: 9 can achieve the purpose of the present disclosure.

In some embodiments, a sequence with only one or a few amino acids substituted, for example, including 1, 2, 3, 4, 5, 6, 7, 8, 9, or 10 conserved amino acid substitutions, with respect to the sequences as set forth in any one of SEQ ID NO: 1 to SEQ ID NO: 9, can also achieve the purpose of the present disclosure. Actually, when the extent of sequence homology between two amino acid sequences is determined or when the combination of CDR1, CDR2, and CDR3 of the single-domain antibody is determined, a person skilled in the art may take into account the so-called "conserved" amino acid substitution. In the case of substitutions, the substitutions are preferably conserved amino acid substitutions. The conserved amino acid substitutions may usually be described as an amino acid substitution in which an amino acid residue is substituted by another amino acid residue having a similar chemical structure, without little or no effect on the function, activity, or other biological properties of the polypeptide. The conserved amino acid substitution is common in the art. For example, the conserved amino acid substitutions are a substitution of one or a few amino acids in the following groups (a)-(d) by another one or a few amino acids in the same groups: (a) polar negatively-charged residues and uncharged amides thereof: Asp, Asn, Glu, and Gln; (b) polar positively-charged residues: His, Arg, and Lys; (c) aromatic residues: Phe, Trp, and Tyr; and (d) aliphatic non-polar or weakly-polar residues: Ala, Ser, Thr, Gly, Pro, Met, Leu, Ile, Val, and Cys. Particularly, the preferred conserved amino acid substitutions are as follows: Asp is substituted by Glu; Asn is substituted by Gln or His; Glu is substituted by Asp; Gln is substituted by Asn; His is substituted by Asn or Gln; Arg is substituted by Lys; Lys is substituted by Arg or Gln; Phe is substituted by Met, Leu, or Tyr; Trp is substituted by Tyr; Tyr is substituted by Phe or Trp; Ala is substituted by Gly or Ser; Ser is substituted by Thr; Thr is substituted by Ser; Gly is substituted by Ala or Pro; Met is substituted by Leu, Tyr, or Ile; Leu is substituted by Ile or Val; Ile is substituted by Leu or Val; Val is substituted by Ile or Leu; or Cys is substituted by Ser. In the present disclosure, preferably, the host cell is a bacterial cell, a fungal cell, or a mammalian cell.

In the present disclosure, the specific antibody is obtained by preparing a target protein and a truncated form of the target protein through the genetic engineering technique, immunizing an Inner Mongolia Alashan Bactrian camel with the obtained antigen protein for multiple times to obtain peripheral blood lymphocytes or spleen cells of the camel, recombining variable region-encoding sequences of a camel-derived antibody into a phage display vector by means of genetic engineering, screening the specific antibody against the antigen protein by the phage display technique. The specific antibody against the antigen protein is further tested for its capability to bind to the antigen and its use in the treatment of Alzheimer's disease and inhibition on tumor.

The foregoing technical solutions are now elaborated in details and described with specific examples:

### Example 1: Preparation of human TREM2 protein:

The human TREM2 protein used herein was obtained through expression and purification by REGENECORE BIOTECH CO., LTD. The encoding sequence of TREM2 was obtained by searching in NCBI with the accession number NM_018965.4, and the amino acid sequence generated by the sequence encoding has an accession number NP_061838.1.

The nucleotide sequence for encoding TREM2 amino acids (from 19 to 174aa) was cloned into the vector pcDNA3.4 by means of gene synthesis. The constructed vector was subjected to Sanger sequencing and aligned with the original sequence. After confirmed, the recombinant plasmid was batch extracted for removal of endotoxins, and transfected into suspension 293F to express the target protein, followed by purification. The purity reached 90% or more, which meets the requirements for animal immunization.

### Example 2: Construction of single-domain antibody library against TREM2 protein:

1 mg of recombinant human TREM2 protein purified in Example 1 was mixed with an equal volume of complete Freund's adjuvant to immunize an Inner Mongolia Alashan Bactrian camel once a week for a total of seven consecutive immunizations. Except for the first immunization, the other six animal immunizations were conducted with 1 mg of TREM2 protein mixed with an equal volume of incomplete Freund's adjuvant. This immunization process was designed to concentratedly stimulate the camel to produce an antibody against the TREM2 protein.

After the animal immunizations, 150 mL of peripheral blood lymphocytes was drawn from the camel, and RNA was extracted from the cells. The extracted total RNA was used to synthesize cDNA, and VHH (a variable region of heavy chain of an antibody) was amplified through a nested polymerase chain reaction (PCR) using cDNA as a template.

The pMECS vector and VHH fragment were then digested with a restriction endonuclease, respectively, and then the digested fragment and vector were ligated together. The ligated fragment was electrotransformed into the competent cells TG1. A phage display library of the TREM2 protein was constructed, and a library size was to be about 1 x 10⁹. In addition, the correct insertion rate of the target fragment in the library was determined by colony PCR.

The results showed that 28 clones out of the 30 colonies randomly selected from the library can be amplified by PCR into the band of predicted size, and two clones were amplified into incorrect bands, so the correct insertion rate was 28÷30× 100%≈93%.

### Example 3: Screening of single-domain antibody against TREM2 protein:

200 µL of the recombinant TG1 cells in Example 2 were cultured in a 2 × TY medium, and during the culturing, 40 µL of helper phage VCSM13 was added to infect the TG1 cells for culture overnight to amplify the phage. On the next day, the phage was precipitated using PEG/NaCl, and the amplified phage was centrifuged and collected.

500 µg of the TREM2 protein diluted in 100 mM NaHCO₃ of pH 8.3 was coupled to an ELISA plate and left to stand at 4°C overnight, and negative control wells (medium control) were set. On the next day, 200 µL of 3% skimmed milk was added and the plate was blocked at room temperature for 2 h. After blocking, 100 µL of amplified phage library (about 2× 10¹¹ phage particles) was added to react at room temperature for 1 h. After 1 h of the reaction, the plate was washed with PBS plus 0.05% Tween-20 for 15 times to wash away unbound phages.

The phage specifically binding to the TREM2 protein was dissociated using trypsin with a final concentration of 25 mg/mL and used to infect *Escherichia coli* TG1 cells in a logarithmic growth phase. The cells were cultured at 37°C for 1 h to produce phages, which were collected for the next round of screening. The same screening process was repeated for one round for gradual enrichment.

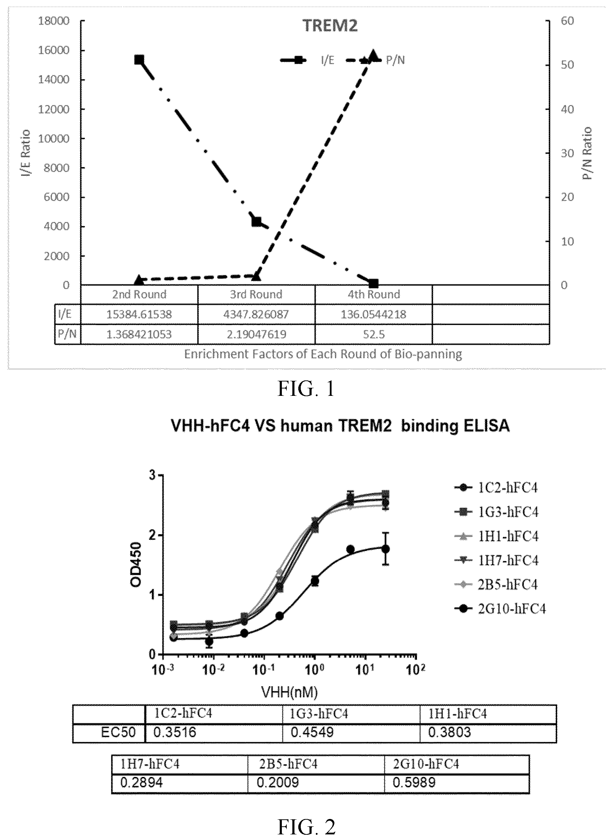

When an enrichment factor reached 10 or more, an enrichment effect was shown in FIG. 1.

In FIG. 1, P/N = the number of monoclonal bacteria grown after TG1 bacteria infected with the phages eluted from the positive wells / the number of monoclonal bacteria grown after TG1 bacteria infected with the phages eluted from the positive wells in the bio-panning, this parameter will gradually increase after enrichment occurs. I/E = the total amount of phages added to the positive wells in each round of bio-panning / the total amount of phages eluted from the positive wells in each round of bio-panning, this parameter will gradually approach 1 after enrichment occurs.

### Example 4: Screening of specific positive clones against TREM2 by phage enzyme-linked immunosorbent assay (ELISA):

The single-domain antibody against the TREM2 protein was screened for four rounds according to the screening method in Example 3, until a enrichment factor of phage against the TREM2 protein reached 10 or more. After the screening, 384 single colonies were picked from screened positive clones and inoculated into a 96 deep-well plate with a 2 × TY medium containing 100 µg/mL ampicillin, while a blank control was set up. After culturing at 37 °C to a logarithmic phase, IPTG was added to a final concentration of 1 mM and the plate was incubated at 28 °C overnight.

Crude antibodies were obtained by osmotic bursting. The TREM2 recombinant proteins were diluted in 100 mM NaHCO₃ of pH 8.3, respectively. An ELISA plate was then coated with 100 µg of the proteins and left at 4°C overnight. 100 µL of the obtained crude antibody extract was transferred to the ELISA plate into which the antigen was added. The plate was incubated at room temperature for 1 h. The unbound antibody was washed away with phosphate buffered saline with Tween (PBST), and 100 µL of Mouse Anti-HA tag Antibody (HRP) (mouse anti-HA horseradish peroxidase-labeled antibody, Thermo Fisher) at 1:2000 dilution was added and incubated at room temperature for 1 h. The unbound antibody was washed away with PBST, and a horseradish peroxidase color development solution was added. After react at 37°C for 15 min, a stopping solution was added. The absorbance value was read at the wavelength of 450 nm on a microplate reader.

When an optical density (OD) value of a sample well is more than five times greater than that of the control well, the sample well is identified as a positive clone well. The bacteria in the positive clone well were transferred to an LB medium containing 100 µg/mL ampicillin under shaking for plasmid extraction and sequencing.

Gene sequences of respective clones were analyzed by using the sequence alignment software VectorNTI. Strains with identical sequences of CDR1, CDR2, and CDR3 were identified as the same clone, while strains with different sequences were identified as different clones. Finally, the single-domain antibodies specifically targeting the TREM2 protein were obtained (including the single-domain antibodies 10B3, 10E12, 10F10, 11D3, 11H2, 12G12W, 1H1, 2B5, 12G10 and the single-domain antibody clones 1C2, 1G3, 1H7, 2G10, 3H3, 4B9, 4F12, 9A8, 9G8, 10B8, 10C7, 10C11, 10E5, 10G6, 10G7, 11B9, 11C4, 11F7, 11H6, 12D7, 12G12F, 12G11, 12G12 with sequences not shown).

The amino acid sequences of the antibodies are of the FR1-CDR1-FR2-CDR2-FR3-CDR3-FR4 structure, which constitutes the entire VHH. The obtained recombinant plasmids for the single-domain antibodies can be expressed in a prokaryotic system, resulting in the single-domain antibody proteins.

The amino acid sequences of the single-domain antibodies 10B3, 10E12, 10F10, 11D3, 11H2, 12G12W, 1H1, 2B5, and 12G10 are sequentially as set forth in SEQ ID NO: 1 to SEQ ID NO: 9, respectively, and the nucleotide sequences thereof are sequentially as set forth in SEQ ID NO: 10 to SEQ ID NO: 18, respectively.

The CDR and FR sequences of the nine single-domain antibodies are shown in Table 1 to Table 7.

**Table 1 CDR1 sequences of the nine single-domain antibodies**

| **Actual clone number** | **CDR1** | **SEQ ID** |
|---|---|---|
| 10F10 | GFAFGNYV | SEQ ID NO: 34 |
| 2B5 | GFAFSNYV | SEQ ID NO: 35 |
| 12G10 | | |
| 10E12 | GFTFSGYY | SEQ ID NO: 36 |
| 11H2 | GLTFSEYY | SEQ ID NO: 37 |
| 1H1 | GRTFSSYV | SEQ ID NO: 38 |
| 11D3 | GRTFTDYA | SEQ ID NO: 39 |
| 10B3 | GRTFTNYA | SEQ ID NO: 40 |
| 12G12W | | |

**Table 2 CDR2 sequences of the nine single-domain antibodies**

| **Actual clone number** | **CDR2** | **SEQ ID** |
|---|---|---|
| 2B5 | IGSQGGAYT | SEQ ID NO: 41 |
| 12G10 | | |
| 10F10 | IGSQGGTYT | SEQ ID NO: 42 |
| 1H1 | ISWSGGIT | SEQ ID NO: 43 |
| 10E12 | ITRGGNT | SEQ ID NO: 44 |
| 11H2 | | |
| 10B3 | VSWSGSST | SEQ ID NO: 45 |
| 11D3 | | |
| 12G12W | | |

**Table 3 CDR3 sequences of the nine single-domain antibodies**

| **Actual clone number** | **CDR3** | **SEQ ID** |
|---|---|---|
| 1H1 | ASGSGSYYRGWEYDY | SEQ ID NO: 46 |
| 10B3 | GVKYFGHSRGGYDY | SEQ ID NO: 47 |
| 11D3 | | |
| 12G12W | | |
| 10E12 | NARRATTYY | SEQ ID NO: 48 |
| 11H2 | | |
| 12G10 | NVVYIGRPN | SEQ ID NO: 49 |
| 10F10 | | |
| 2B5 | NVVYNGRPN | SEQ ID NO: 50 |

**Table 4 FR1 sequences of the nine single-domain antibodies**

| **Actual clone number** | **FR1** | **SEQ ID** |
|---|---|---|
| 10B3 | ESGGGLVQAGESLRLSCAAS | SEQ ID NO: 19 |
| 11D3 | | |
| 12G12W | | |
| 10E12 | ESGGGLVQPGGSLRLSCAAS | SEQ ID NO: 20 |
| 10F10 | | |
| 11H2 | | |
| 1H1 | | |
| 2B5 | | |
| 12G10 | | |

**Table 5 FR2 sequences of the nine single-domain antibodies**

| **Actual clone number** | **FR2** | **SEQ ID** |
|---|---|---|
| 1H1 | MGWFRQAPGKEREFVAA | SEQ ID NO: 21 |
| 10B3 | MGWFRQAPGKEREFVAG | SEQ ID NO: 22 |
| 11D3 | | |
| 12G12W | | |
| 12G10 | MHWYRQAPEKERELVAI | SEQ ID NO: 23 |
| 10F10 | | |
| 2B5 | MHWYRQAPEKERELVAL | SEQ ID NO: 24 |
| 10E12 | MSWVRQVPGKARELVAG | SEQ ID NO: 25 |
| 11H2 | | |

**Table 6 FR3 sequences of the nine single-domain antibodies**

| **Actual clone number** | **FR3** | **SEQ ID** |
|---|---|---|
| 10B3 | DYADSVKGRFTISTDNAKNTVYLQINNLKPEDTAVYIC | SEQ ID NO: 26 |
| 11D3 | DYADSVKGRFTISTDNAKNTVYLQMTSLKPEDTAVYIC | SEQ ID NO: 27 |
| 12G12W | | |
| 10F10 | NYADSVKGRFTISRDNAKNMVYLQMNNLKPEDTAVYYC | SEQ ID NO: 28 |
| 12G10 | NYAASVKGRFTISRDNAKNMVYLQMNNLKPEDTAVYYC | SEQ ID NO: 29 |
| 2B5 | NYAGSVKGRFTISRDNTKNMVYLQMNNLKPEDTAVYYC | SEQ ID NO: 30 |
| 10E12 | YYADSVKGRFTISRDNAKNTVYLQMNNLKPEDTTVYYC | SEQ ID NO: 31 |
| 11H2 | | |
| 1H1 | YYGDSVKGRFTISRDNAKNTVFLQMNSLKPEDTAVYYC | SEQ ID NO: 32 |

**Table 7 FR4 sequences of the nine single-domain antibodies**

| **Actual clone number** | **FR4** | **SEQ ID** |
|---|---|---|
| 10B3 | WGQGTQVTVSS | SEQ ID NO: 33 |
| 10E12 | | |
| 10F10 | | |
| 11D3 | | |
| 11H2 | | |
| 12G12W | | |
| 1H1 | | |
| 2B5 | | |
| 12G10 | | |

### Example 5: Purification and expression of single-domain antibody specific to TREM2 protein in host bacteria Escherichia coli

The plasmids (pMECS-VHH) of different clones obtained through sequencing in Example 4 were electrotransformed into *Escherichia coli* HB2151, and then coated on an Lb+amp+glucose culture plate, that is, a plate containing ampicillin and glucose, for culture at 37°C overnight. A single colony was picked and inoculated into 5 mL of LB liquid medium containing ampicillin, for culture at 37°C on a shaker overnight.

1 mL of the culture that had been incubated overnight was inoculated into 330 mL of TB liquid medium, for culture at 37°C on a shaker. When the OD600 value reached 0.6-0.9, 1M isopropyl-beta-D-thiogalactopyranoside (IPTG) was added for culture at 28°C on a shaker overnight. *Escherichia coli* were collected by centrifugation. A crude antibody extract was obtained by the osmotic bursting.

The antibody was purified by nickel-column affinity chromatography.

### Example 6: Construction of eukaryotic expression vectors for Fc Fusion antibodies of anti-TREM2 single-domain antibody

(1) The target sequence obtained in Example 4 was subcloned into a eukaryotic expression vector. The antibody screened out in Example 4 was subjected to Sanger sequencing to obtain its nucleotide sequence.
(2) A recombinant eukaryotic expression vector is obtained by synthesizing the respective nucleotide sequences (SEQ ID NO: 10 to SEQ ID NO: 18 and nucleotide sequences of other single-domain antibody clones with sequences not shown) into the vector RJK-V4-3 or RJK-V4-hFC1 designed and modified as described in Example 10 by REGENECORE BIOTECH CO., LTD, by means of sequence synthesis.
(3) The recombinant eukaryotic expression vector constructed in step (2) was transformed into DH5α *Escherichia coli* and cultured. The plasmid was extracted, and endotoxin was removed.
(4) The extracted plasmid was then subjected to sequencing for identification.
(5) The confirmed recombinant vector was prepared for subsequent transfection and expression in eukaryotic cells. The Fc protein of VHH was expressed by the method in Example 7 or 8, and then the foregoing antibody was purified by the method in Example 9.

### Example 7: Expression of single-domain antibody against TREM2 protein in ExpiCHO-S cells in suspension

(1) Three days before transfection, ExpiCHO-S^{™} cells at 2.5 × 10⁵ cells/mL were passaged and expanded. The cells in a volume by calculation were transferred into a 500 mL shake flask containing 120 mL (final volume) of fresh pre-warmed ExpiCHO^{™} expression medium. The cells were incubated to a concentration of about 4 × 10⁶ to 6 × 10⁶ live cells/mL.
(2) On the day before transfection, ExpiCHO-S^{™} cells were diluted to a concentration of 3.5 × 10⁶ live cells/mL, and cultured overnight.
(3) On the day of transfection, the cell density and percentage of live cells were determined. Before transfection, the cell density reached about 7 × 10⁶ to 10 × 10⁶ live cells/mL.
(4) The cells were diluted to 6 × 10⁶ live cells/mL with the fresh ExpiCHO^{™} expression medium pre-warmed to 37 °C. The cells in a volume by calculation were transferred into a 500 mL shake flask containing 100 mL (final volume) of freshly pre-warmed ExpiCHO^{™} expression medium.
(5) ExpiFectamine^{™}CHO reagent was mixed by gentle inversion, and ExpiFectamine^{™}CHO reagent was diluted with 3.7 mL of OptiPRO^{™} medium, and shaken or mixed uniformly.
(6) The plasmid DNA was diluted with 4 mL of refrigerated OptiPRO^{™} medium, and shaken and mixed uniformly.
(7) ExpiFectamine CHO/plasmid DNA (the plasmid DNA was the eukaryotic expression vector for the Fc fusion antibody of the anti-TREM2 single-domain antibody obtained in Example 6) complex was incubated at room temperature for 1 to 5 minutes, and then gently added into the prepared cell suspension, during which the shake flask was gently shaken.
(8) The cells were cultured by shaking at 37°C in 8% CO₂ in humidified air.
(9) On day 1 after transfection (18 to 22 hours later), 600 µL of ExpiFectamine^{™}CHO Enhancer and 24 mL of ExpiCHO feed were added.
(10) About eight days after transfection (cell viability below 70%), a supernatant was collected.

### Example 8: Expression of single-domain antibody against TREM2 protein in 293F cells in suspension

Experimental procedure of expression of the recombinant single-domain antibody (taking expression in a 500 mL shake flask as an example):
(1) Three days before transfection, 293F cells at 2.5 × 10⁵ cells/mL were passaged and expanded. The cells in a volume by calculation were transferred into a 500 mL shake flask containing 120 mL (final volume) of freshly pre-warmed OPM-293 CD05 Medium. The cells were incubated to a concentration of about 2 × 10⁶ to 3 × 10⁶ live cells/mL.
(2) On the day of transfection, the cell density and percentage of live cells were determined. Before transfection, the cell density reached about 2 × 10⁶ to 3 × 10⁶ live cells/mL.
(3) The cells were diluted to 1 × 10⁶ live cells/mL with the pre-warmed OPM-293 CD05 Medium. The cells in a volume by calculation were transferred into a 500 mL shake flask containing 100 mL (final volume) of freshly pre-warmed medium.
(4) Polyethyleneimine (PEI; 1 mg/mL) reagent was diluted with 4 mL of Opti-MEM medium, and uniformly mixed by shaking or pipetting up and down. The plasmid DNA (the plasmid DNA was the eukaryotic expression vector for the Fc fusion antibody of the anti-TREM2 single-domain antibody obtained in Example 6) was diluted with 4 mL of Opt-MEM medium, uniformly mixed by shaking, filtered through a 0.22 µm filter head, and then incubated at room temperature for 5 min.
(5) The diluted PEI reagent was added to the diluted DNA and mixed by inversion. The PEI/plasmid DNA complex was incubated at room temperature for 15 to 20 minutes, and then gently added into the prepared cell suspension, during which the shake flask was gently shaken.
(6) The cells were cultured by shaking at 37°C in 5% CO₂ at 120 rpm.
(7) 5 mL of OPM-CHO PFF05 supplement was added at 24 h and 72 h after transfection.
(8) About seven days after transfection (cell viability below 70%), a supernatant was collected.

### Example 9: Purification of single-domain antibody against TREM2 protein

(1) The protein expression supernatant obtained in Example 7 or 8 was filtered through a 0.45 µm disposable filter head to remove insoluble impurities.
(2) The filtrate was purified by affinity chromatography using a protein purifier, with a Protein A-conjugated agarose filler based on the capability of binding human Fc to Protein A.
(3) The filtrate was allowed to flow through a Protein A pre-packed column at a flow rate of 1 mL/min, during which the target protein in the filtrate bound to the filler.
(4) Impurity proteins bound to the column were washed away with a low-salt buffer and a high-salt buffer.
(5) The target protein bound to the column was eluted with a low-pH buffer.
(6) The eluate was quickly added to the Tris-HCl solution at pH 9.0 for neutralization.
(7) After dialysis, the neutralized protein solution was subjected to SDS-PAGE analysis, and then stored at low temperature for later use when it was determined that the protein purity was 95% or more and the concentration was 0.5 mg/mL or more.

### Example 10: Construction of eukaryotic expression vector for single-domain antibody

The mentioned universal target vector RJK-V4-3 for nanobodies is obtained from REGENECORE BIOTECH CO., LTD by fusing the commercial vector pCDNA3.4 from invitrogen (link to vector information: https://assets.thermofisher.com/TFS-Assets/LSG/manuals/pcdna3_4_topo_ta_cloning_kit_man.pdf) with the Fc region of the heavy-chain encoding sequence of human IgG4 . In other words, the vector contains hinge, CH2, and CH3 regions of the IgG4 heavy chain. The specific modification scheme was as follows:
(1) The restriction sites XbaI and AgeI in pcDNA3.4 were selected.
(2) Multiple cloning sites (MCSs) and 6 × His tag were introduced at the 5' end and 3' end of the Fc fragment-encoding sequence by overlapping PCR, respectively.
(3) The fragment was amplified by PCR using a pair of primers with the restriction sites Xbal and AgeI, respectively.
(4) The pcDNA3.4 and the recombinant DNA fragment in (3) were digested using the restriction endonucleases XbaI and AgeI, respectively.
(5) The digested vector and the fragment to be inserted were ligated under the action of T4 ligase. Then, the ligated product was transformed into *Escherichia coli,* amplified, and sequenced for identification, to obtain the recombinant plasmid.

The mentioned universal target vector RJK-V4-hFC1 for nanobodies is obtained from REGENECORE BIOTECH CO., LTD by fusing the commercial vector pCDNA3.4 from Invitrogen (link to vector information: https://assets.thermofisher.com/TFS-Assets/LSG/manuals/pcdna3_4_topo_ta_cloning_kit_man.pdf) with the Fc region of the heavy-chain encoding sequence of human IgG1. In other words, the vector contains hinge, CH2, and CH3 regions of the IgG1 heavy chain. The specific modification scheme was as follows:
(1) The restriction sites XbaI and AgeI on pcDNA3.4 were selected.
(2) Multiple cloning sites (MCSs) and 6×His tags were introduced at the 5' end and 3' end of the Fc fragment-encoding sequence by overlapping PCR, respectively.
(3) The fragment was amplified by PCR using a pair of primers with the restriction sites Xbal and AgeI, respectively.
(4) The pcDNA3.4 and the recombinant DNA fragment in (3) were digested using the restriction endonucleases XbaI and AgeI, respectively.
(5) The digested vector and the fragment to be inserted were ligated under the action of T4 ligase. Then, the ligated product was transformed into *Escherichia coli,* amplified, and sequenced for identification, to obtain the recombinant plasmid.

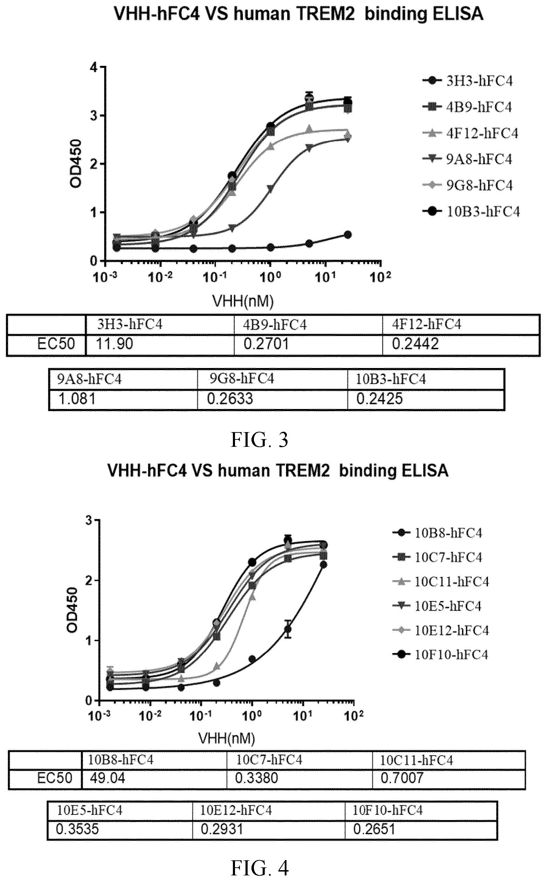

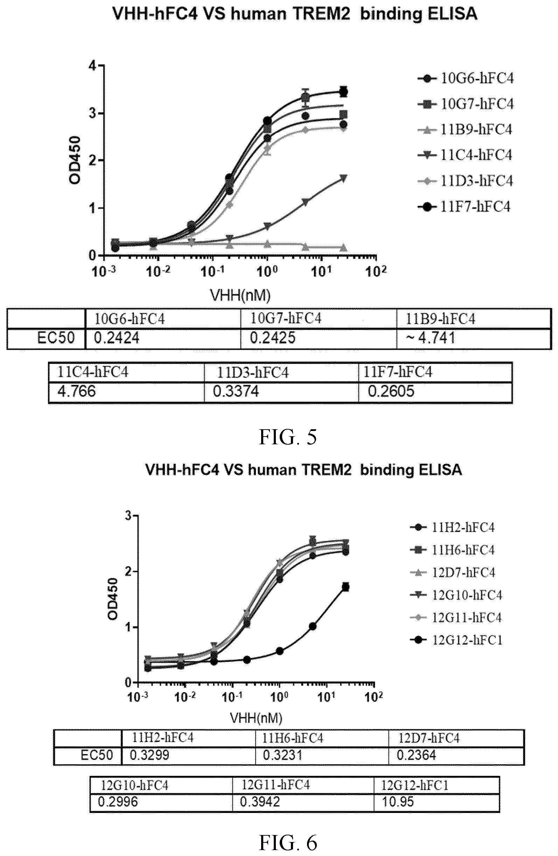

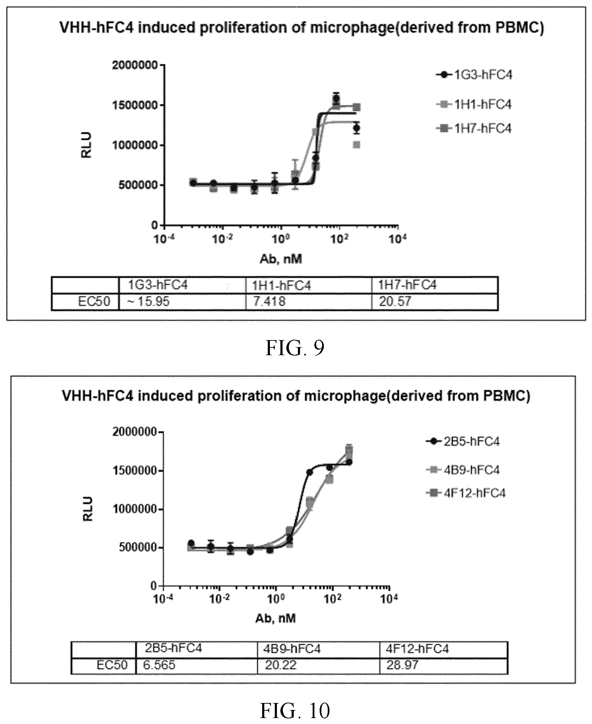

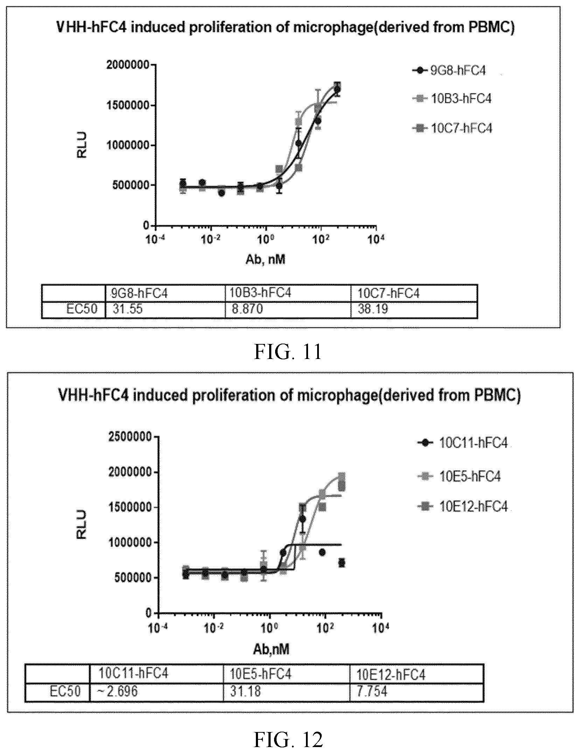

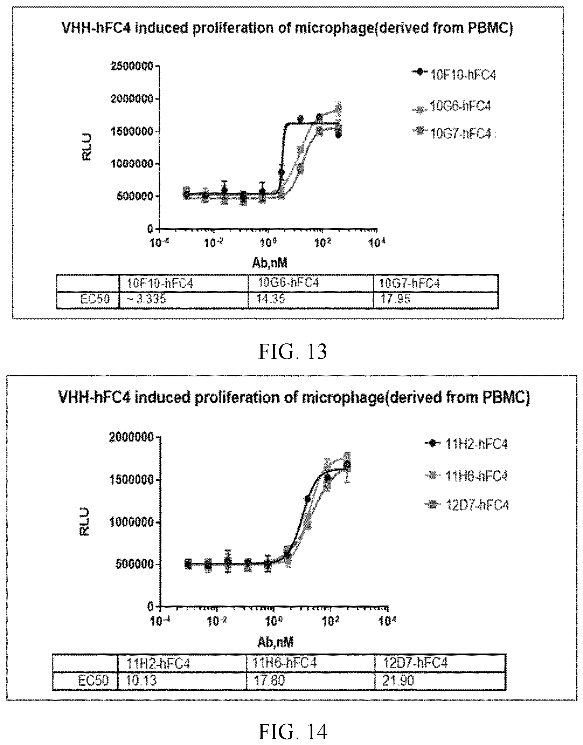

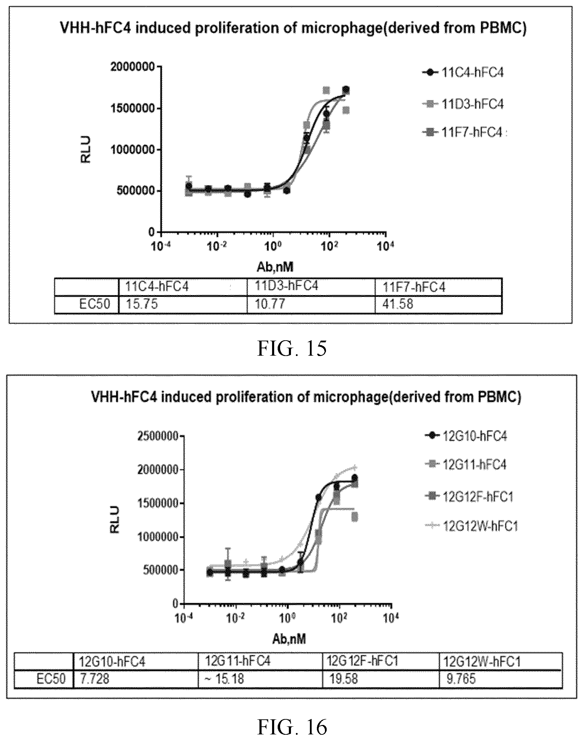

In FIG. 2 to FIG. 16, the name with hFC1 is obtained by cloning the corresponding single-domain antibody sequence into RJK-V4-hFC1 (for example, 12G12W-hFC1 is obtained by cloning the nucleotide sequence corresponding to the single-domain antibody 12G12W into RJK-V4-hFC1), and the name with hFC4 is obtained by cloning the corresponding single-domain antibody sequence into RJK-V4-3 (for example, 11H2-hFC4 is obtained by cloning the nucleotide sequence corresponding to the single-domain antibody 11H2 into RJK-V4-3).

### Example 11: Expression and purification of tool antibody (Tab1) targeting human TREM2

Herein, Tab1 is AL2p-58 huIgG PSEG (AT.1FM) (WO 2021/203030 A2).

The searched sequence was entrusted to GENERAL BIOL (Anhui) Co., Ltd for codon optimization in a mammalian cell expression system, and cloned into pcDNA3.1 vector. After screening for resistance, plasmid-positive bacteria were selected for amplification, and plasmids were extracted using the plasmid midiprep kit (Macherey Nagel, Cat#740412.50). 100 µg of plasmids (40 µg of heavy chains + 60 µg of light chains) were added per 100 mL of cells, and transiently expressed in 293F cells (medium: FreeStyle 293 Expression medium, Thermo, Cat#12338026 + F-68, Thermo, Cat#24040032) with PEI. At 6 to 24 h after transfection, 5 vol% of 10% Peptone (Sigma, Cat#P0521-100G) was added, and then the cells were cultured in 8% CO₂ at 130 rpm for about 7-8 days. An expression supernatant was collected when the cell viability decreased to 50%, and purified using the Protein A (GE, Cat#17-5438-02) gravity chromatography column. After dialysis with PBS, the concentration was determined using Nanodrop, the purity was identified by SEC, and the binding capacity was verified by indirect ELISA.

The Tab obtained by this method had a concentration of no less than 2 mg/mL and a purity of greater than 95%.

### Example 12: Determination of antigen-binding dose-response curve of antibody

This example was implemented according to the standard operation procedure of enzyme-linked immunosorbent assay (ELISA).
(1) 50 µL of 1 µg/mL TREM2 protein was coated and left at 4°C overnight.
(2) The plate was washed; and 200 µL of 5% milk was added and the plate was blocked at 37°C for 2 h.
(3) VHH-hFc was diluted to 2 µg/mL, and then the antibody was diluted in a 5-fold gradient for a total of eight concentration gradients. The VHH-hFc herein was obtained by purifying (according to Example 9) the Fc fusion antibody (expressed in 293F cells) of the single-domain antibody against the TREM2 protein prepared in Example 8. In addition, hIgG and Tab1 controls were set separately; and Tab1 was prepared in Example 11.
(4) The plate was washed; and 50 µL of the single-domain antibody obtained through dilution in step (3) was added in duplicate, and incubated at 37°C for 1 h.
(5) The plate was washed; and 50 µL of HRP-Goat anti hIgG secondary antibody was added, and incubated at 37 °C for 30 min.
(6) The plate was washed (for several times); and 50 µL of 3,3',5,5'-Tetramethylbenzidine (TMB) restored to room temperature was added and reacted at room temperature in the dark for 15 min.
(7) 50 µL of stopping solution (1N HCl) was added. The value was read on a microplate reader and recorded.
(8) The curve was plotted and EC50 was calculated. As shown in FIG. 2 to FIG. 7, hIgG is an isotype control, which is an immunoglobulin molecule that binds to no target and is commercially available.

As can be seen from FIG. 2 to FIG. 7, all the nine single-domain antibodies of the present disclosure have excellent binding capacity and specificity to the TREM2 protein.

### Example 13: Stimulation of antibody (eukaryotic sample) on macrophage proliferation

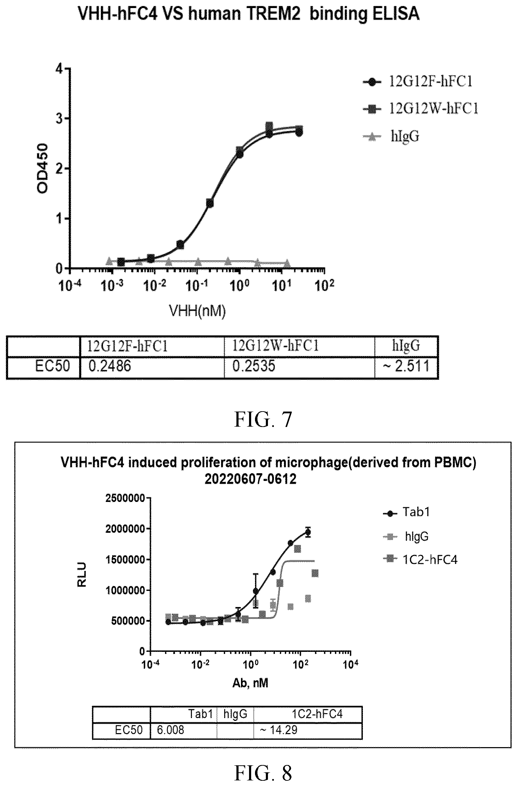

The following operations were performed according to the methods common to a person skilled in the art:
The cells used were macrophages induced from PBMC. The steps were as follows: CD14+ cells sorted by magnetic activated cell sorting (MACS) were subjected to macrophage colony-stimulating factor (M-CSF)-induced culture for 11 days to obtain macrophages. The thawed macrophages passaged for 3-4 times were spread into a 96-well plate at 10,000 cells per well. hIgG, the Tab1, and the Fc fusion protein of the single-domain antibody provided in the example (obtained by purifying (according to Example 9) the Fc fusion antibody of the single-domain antibody against the TREM2 protein prepared in Example 8) were prepared into a 10 µg/mL solution and diluted in a 5-fold gradient, respectively, and the antibody solutions were added to the cell culture wells in an equal volume of the cell culture solution. After incubation for 120 h, the cell viability was detected using the luminescent cell viability assay kit. The EC50 concentrations of the survival of macrophages stimulated by different single-domain antibodies were calculated based on the assay results. The results are shown in FIG. 8 to FIG. 16.

The foregoing descriptions are merely preferred examples of the present disclosure and are not intended to limit the present disclosure. A person skilled in the art may make various modifications and changes of the present disclosure. Any modification, equivalent replacement, or improvement made within the spirit and principle of the present disclosure shall fall within the protection scope of the present disclosure.

## Claims

1. An anti-TREM2 single-domain antibody, wherein the single-domain antibody consists of a heavy chain comprising a heavy chain CDR1, a heavy chain CDR2, and a heavy chain CDR3; and amino acid sequences of the heavy chain CDR1, the heavy chain CDR2, and the heavy chain CDR3 are one of the following (1) to (8):
(1) CDR1 as set forth in SEQ ID NO: 34, CDR2 as set forth in SEQ ID NO: 42, and CDR3 as set forth in SEQ ID NO: 49;
(2) CDR1 as set forth in SEQ ID NO: 35, CDR2 as set forth in SEQ ID NO: 41, and CDR3 as set forth in SEQ ID NO: 50;
(3) CDR1 as set forth in SEQ ID NO: 35, CDR2 as set forth in SEQ ID NO: 41, and CDR3 as set forth in SEQ ID NO: 49;
(4) CDR1 as set forth in SEQ ID NO: 36, CDR2 as set forth in SEQ ID NO: 44, and CDR3 as set forth in SEQ ID NO: 48;
(5) CDR1 as set forth in SEQ ID NO: 37, CDR2 as set forth in SEQ ID NO: 44, and CDR3 as set forth in SEQ ID NO: 48;
(6) CDR1 as set forth in SEQ ID NO: 38, CDR2 as set forth in SEQ ID NO: 43, and CDR3 as set forth in SEQ ID NO: 46;
(7) CDR1 as set forth in SEQ ID NO: 39, CDR2 as set forth in SEQ ID NO: 45, and CDR3 as set forth in SEQ ID NO: 47; and
(8) CDR1 as set forth in SEQ ID NO: 40, CDR2 as set forth in SEQ ID NO: 45, and CDR3 as set forth in SEQ ID NO: 47.

2. The anti-TREM2 single-domain antibody according to claim 1, wherein the heavy chain further comprises a framework region FR; the framework region FR comprises amino acid sequences of FR1, FR2, FR3, and FR4; and amino acid sequences of the framework region FR are as follows respectively:
FR1 as set forth in SEQ ID NO: 19 or SEQ ID NO: 20, or an FR1 variant comprising at most five amino acid substitutions with respect to the FR1;
FR2 set forth in any one of SEQ ID NO: 21 to SEQ ID NO: 25, or an FR2 variant comprising at most five amino acid substitutions with respect to the FR2;
FR3 as set forth in any one of SEQ ID NO: 26 to SEQ ID NO: 32, or an FR3 variant comprising at most five amino acid substitutions with respect to the FR3; and
FR4 as set forth in SEQ ID NO: 33, or an FR4 variant comprising at most five amino acid substitutions with respect to the FR4.

3. An anti-TREM2 single-domain antibody, wherein an amino acid sequence of the single-domain antibody is as set forth in any one of SEQ ID NO: 1 to SEQ ID NO: 9, or an amino acid sequence of the single-domain antibody has at least 80% sequence homology with any one of SEQ ID NO: 1 to SEQ ID NO: 9.

4. An Fc fusion antibody or a humanized antibody of the anti-TREM2 single-domain antibody according to any one of claims 1 to 3.

5. A recombinant protein comprising the anti-TREM2 single-domain antibody according to any one of claims 1 to 3.

6. A nucleotide molecule for encoding the anti-TREM2 single-domain antibody according to any one of claims 1 to 3, wherein a nucleotide sequence of the nucleotide molecule is as set forth in any one of SEQ ID NO: 10 to SEQ ID NO: 18, or has at least 80% sequence homology with any one of SEQ ID NO: 10 to SEQ ID NO: 18.

7. An expression vector, comprising a nucleotide molecule for encoding the anti-TREM2 single-domain antibody according to any one of claims 1 to 3 or the Fc fusion antibody or the humanized antibody according to claim 4, or the nucleotide molecule according to claim 6.

8. A host cell, capable of expressing the anti-TREM2 single-domain antibody according to any one of claims 1 to 3 or the Fc fusion antibody or the humanized antibody according to claim 4, or comprising the expression vector according to claim 7.

9. A pharmaceutical composition, wherein the pharmaceutical composition comprises the anti-TREM2 single-domain antibody according to any one of claims 1 to 3 and a pharmaceutically acceptable carrier.

10. A medicament for treatment of a neurodegenerative disease or a tumor, comprising the anti-TREM2 single-domain antibody according to any one of claims 1 to 3 as an active ingredient.

11. Use of the anti-TREM2 single-domain antibody according to any one of claims 1 to 3 or the pharmaceutical composition according to claim 9 in preparation of a medicament for treatment of a disease.

12. The use according to claim 11, wherein the disease is a neurodegenerative disease or a tumor.
